(19) Europäisches Patentamt
European Patent Office
Office européen des brevets

(11) EP 1 315 824 B1

(12) # EUROPÄISCHE PATENTSCHRIFT

(45) Veröffentlichungstag und Bekanntmachung des Hinweises auf die Patenterteilung:
**15.11.2006 Patentblatt 2006/46**

(21) Anmeldenummer: **01971983.0**

(22) Anmeldetag: **30.08.2001**

(51) Int Cl.:
***C12P 13/02*** *(2006.01)* ***C12P 7/40*** *(2006.01)*
***C12N 1/20*** *(2006.01)* ***C12N 9/14*** *(2006.01)*
***C12N 9/88*** *(2006.01)*

(86) Internationale Anmeldenummer:
**PCT/EP2001/010025**

(87) Internationale Veröffentlichungsnummer:
**WO 2002/018612 (07.03.2002 Gazette 2002/10)**

(54) **VERFAHREN ZUR ENZYMATISCHEN UMSETZUNG VON VERBINDUNGEN MIT MINDESTENS EINER NITRILFUNKTION UND/ODER MINDESTENS EINER AMIDFUNKTION**

METHOD FOR AN ENZYMATIC REACTION OF COMPOUNDS HAVING AT LEAST ONE NITRILE FUNCTION AND/OR AT LEAST ONE AMIDE FUNCTION

PROCEDE DE MISE EN REACTION ENZYMATIQUE DE COMPOSES POSSEDANT AU MOINS UNE FONCTION NITRILE ET / OU AU MOINS UNE FONCTION AMIDE

(84) Benannte Vertragsstaaten:
**AT BE CH CY DE DK ES FI FR GB GR IE IT LI LU MC NL PT SE TR**

(30) Priorität: **30.08.2000 DE 10042835**

(43) Veröffentlichungstag der Anmeldung:
**04.06.2003 Patentblatt 2003/23**

(73) Patentinhaber: **BASF Aktiengesellschaft**
**67056 Ludwigshafen (DE)**

(72) Erfinder:
- **HAUER, Bernhard**
  **67136 Fussgönheim (DE)**
- **PRESSLER, Uwe**
  **67165 Waldsee (DE)**
- **RESS-LÖSCHKE, Marion**
  **69221 Dossenheim (DE)**
- **SYLDATK, Christoph**
  **70565 Stuttgart (DE)**
- **CHRISTIAN, Hans-Jürgen**
  **13353 Berlin (DE)**
- **PIETZSCH, Markus**
  **38110 Braunschweig (DE)**

(56) Entgegenhaltungen:
**EP-A- 0 204 555** **DE-C- 4 313 649**
**US-A- 5 030 571**

- **DATABASE CA [Online] CHEMICAL ABSTRACTS SERVICE, COLUMBUS, OHIO, US; ACHARYA, A. ET AL: "Studies on utilization of acetonitrile by Rhodococcus erythropolis A10" retrieved from STN Database accession no. 126:340847 XP002185545 & WORLD J. MICROBIOL. BIOTECHNOL. (1997), 13(2), 175-178,**
- **EFFENBERGER F ET AL: "Chemo- and enantioselective hydrolysis of nitriles and acid amides, respectively, with resting cells of Rhodococcus sp. C3II and Rhodococcus erythropolis MP50" JOURNAL OF BIOTECHNOLOGY, ELSEVIER SCIENCE PUBLISHERS, AMSTERDAM, NL, Bd. 60, Nr. 3, 26. Februar 1998 (1998-02-26), Seiten 165-174, XP004128040 ISSN: 0168-1656**
- **DATABASE CA [Online] CHEMICAL ABSTRACTS SERVICE, COLUMBUS, OHIO, US; LANGDAHL, BJARNE R. ET AL: "Nitrile hydrolysis by Rhodococcus erythropolis BL1, an acetonitrile-tolerant strain isolated from a marine sediment" retrieved from STN Database accession no. 124: 111995 XP002185546 & MICROBIOLOGY (READING, U. K.) (1996), 142(1), 145-54,**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 315 824 B1

## Beschreibung

Nitrilverbindungen sind in der Natur weit verbreitet. In allen bisher untersuchten Organismenklassen kommen Vertreter mit der Eigenschaft der Synthese von Nitrilverbindungen vor.

In der chemischen Synthese werden Nitrile unter anderen als hydrophiles und leicht flüchtiges Lösungsmittel eingesetzt, wie zum Beispiel Acetonitril, Propionitril und Benzylcyanid als Ausgangsmaterial für Benzylharze. Acrylonitril und Adiponitril werden in großen Mengen zur Herstellung von Polymeren wie Latex und Nylon verwendet. Auch als Schutzgruppe in der chemischen Synthese wird Blausäure häufig verwendet und fällt daher als Abfallstoff an.

Das Nitril der Propionsäure wird unter anderem technisch durch die Alkylierung von Cyaniden (Kolbesynthese) oder die Dehydratisierung von Propionamid hergestellt.

In der Natur werden Nitrilverbindungen zumeist durch Mikroorganismen abgebaut. Die Umsetzung von Nitrilverbindungen durch lebende mikrobielle Zellen erfolgt auf zwei unterschiedlichen Wegen.

Eine Nitrilase (E.C. 3.5.5.1) kann die Umsetzung von Nitrilen zur Säure ohne die Freisetzung des Zwischenproduktes Amid direkt in einem Einschrittverfahren katalysieren.

Ein Nitrilhydratase-Amidase-System ist dagegen eine Zweienzymreaktion, bei der die Nitrilhydratase (E.C. 4.2.1.84) das Nitril zum Carbonsäureamid verseift. Dieser Schritt ist eine Addition von Wasser, weshalb die Nitrilhydratasen nicht unter die Hydrolasen, sondern unter die Lyasen eingegliedert werden.

Das Carbonsäureamid wird dann durch eine Amidase (E.C. 3.5.1.4) in einem zweiten Schritt in die korrespondierende Säure und Ammonium umgewandelt. Die unterschiedlichen Reaktionswege sind in folgender Abbildung dargestellt.

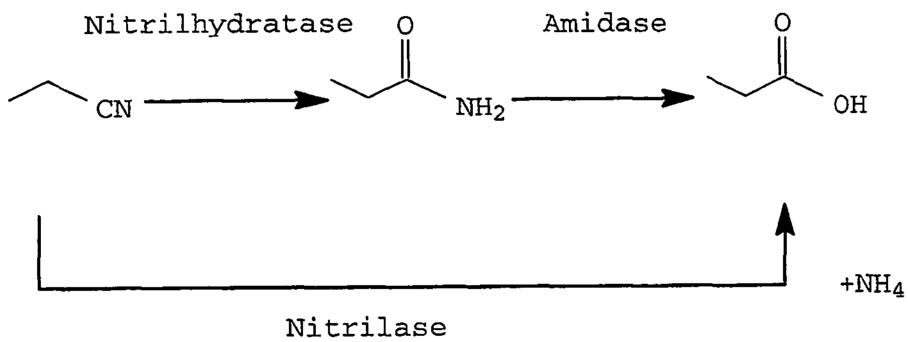


Nitrilhydrataseaktivitäten sind in der Familie der Actinomyceten weit verbreitet, auch gewisse niedere Pilze können solche Verbindungen verwerten. Besonders die Gattung Rhodococcus, mycelartig wachsende Bodenbakterien, können mit Nitrilen als alleiniger Kohlenstoff- und Stickstoffquelle wachsen. Aber auch Vertreter der Gattung Corynebacterium, Brevibacterium und Agrobacterium besitzen Nitrilhydratasen zur Verstoffwechslung von natürlich auftretenden Polymeren mit Nitrilgruppen. Auch unter den Vertretern der Gattung Pseudomonas sind Stämme mit nitrilhydrolysierenden Eigenschaften bekannt.

US 4,421,855 beschreibt ein Verfahren zur Herstellung von Acrylamid, ausgehend von Acrylnitril, bei dem ein immobilisierter Mikroorganismus mit Nitrilhydrataseaktivität eingesetzt wird. Bei diesem Verfahren wird der Mikroorganismus in einem Polyacrylamidgel immobilisiert.

EP 0 252 564 B1 beansprucht ein Rhodococcus Sp. NCIB 122 18 sowie ein Verfahren zur Herstellung von 2,6-Difluorbenzamid unter Einsatz des Bakteriums der Gattung Rhodococcus.

WO 95/04828 beschreibt Enzyme mit einer Nitrilhydrataseaktivität sowie Mikroorganismen, die diese Enzyme überexprimieren.

EP 0 204 555 B1 beschreibt ein Verfahren, bei dem ein Nitril mit einem Mikroorganismus (Rhodococcus Sp. AK-32, Rhodococcus Sp. AK-33) zum entsprechenden Amid umgesetzt wird.

EP 133 927 A1 beschreibt die Umsetzung von Nitrilen zu den korrespondierenden Amiden durch geeignete Mikroorganismen. Die Aktivität dieser Mikroorganismen ist lichtinduzierbar. Beispiele für geeignete Mikroorganismen sind grampositive Bakterien der Gattung Coryne-Bakterium, Nocardia, Bacillus, Bakteridium, Mikrococcus und Brevi-Bakterium.

DE 25 56 701.5 (US 4,001,081) beschreibt ein Verfahren zur Herstellung von Amiden durch Hydrolyse der entsprechenden Nitrile, bei dem man Bakterien aus den Arten Bacillus, Bakteridium, Mikrococcus und Brevi-Bakterium einsetzt.

US 5,030,571 beansprucht den Stamm Rhodococcus erythropolis NCIB 122 73 sowie seinen Arylacylamidase-produzierenden Mutanten.

EP 0 326 482 B1 beschreibt ein Verfahren zur Herstellung von S(+)-Enantiomeren von 2-Arylalkansäuren, bei dem die entsprechenden Amide durch Mikroorganismen oder Enzyme, die aus Mikroorganismen gewonnen werden, umgesetzt werden. Die Mikroorganismen, deren Amidaseaktivität man sich hierbei zu Nutze macht, sind ausgewählt unter den Brevi-Bakterien und CoryneBakterien.

EP 0 344 044 B1 beschreibt ein Verfahren zur Herstellung von R(+) Enantiomeren der 2-Aryloxy- oder 2-Arylthioalkansäuren ausgehend von den entsprechenden Amiden durch Umsetzung mit Corynebakterium oder Brevibakterium.

US 4,629,700 beschreibt ein Verfahren, bei dem aromatische Polynitrile von Mikroorganismen mit Nitrilaseaktivität (Rhodococcen) umgesetzt werden.

EP 0 348 901 B1 beschreibt ein Verfahren zur Herstellung von optisch aktiven $\alpha$-substituierten organischen Säuren. Bei diesem Verfahren wird ein racemisches $\alpha$-substituiertes Nitril oder Amid mit einem Mikroorganismus aus der Gattung Alcaligenes, Pseudomonas, Rodopseudomonas, Corynebakterium sp., Acinetobacter, Bacillus, Microbakterium, Rhodococcus und Candida umgesetzt. Des weiteren wird eine Nitrilase aus Acinetobacter sp. Stamm AK 226 beansprucht, die ein racemisches $\alpha$-substituiertes Nitril in eine optisch aktive $\alpha$-substituierte organische Säure überführen kann.

DE 24 44 849 C2 beschreibt ein Verfahren zur Herstellung organischer Säuren durch Hydrolyse der entsprechenden Nitrile. Bei diesem Verfahren werden definierte Bakterienstämme (grampositiv) eingesetzt.

DE 43 13 649 C1 beschreibt einen Mikroorganismus Rhodococcus erythropolis DSM 7529 sowie ein Verfahren zur mikrobiellen Herstellung eines Nitrilhydratase-/Amidase-Enzymkomplexes für den Abbau organischer Nitrile. Der beanspruchte Mikroorganismus überführt die Nitrilgruppen tragenden organischen Zwischenprodukte in die entsprechenden Amide bzw. Carbonsäurederivate. Dazu benötigt der Mikroorganismus als Induktoren Nitrile, Lactame oder Ionen von Nebengruppenelementen.

EP 0 473 328 A2 beansprucht ein Verfahren zur Herstellung optisch aktiver 2-substituierter Carbonsäuren. Dazu wird das racemische 2-substituierte Nitril mit Mikroorganismen umgesetzt, die in der Lage sind, die Nitrilgruppe zur Carbonsäuregruppe umzusetzen. Unter den erwähnten Mikroorganismen befindet sich auch ein Rhodococcus erythropolis (IFO 123 20).

EP 0 330 529 B1 beansprucht ein Verfahren zur Herstellung von S-Enantiomeren der 2-Arylpropionsäuren, bei dem ein Mikroorganismus aus der Gattung der Brevibakterien und Corynebakterien eingesetzt werden.

EP 0 444 640 B1 beansprucht ein Verfahren zur Erzeugung einer erhöhten Menge an Nitrilaseaktivität aus einem Mikrobenstamm von Rhodococcus. Hierbei werden Lactamverbindungen als Induktoren für die Nitrilaseaktivität eingesetzt.

Nachteilig an den aus dem Stand der Technik bekannten Verfahren ist, daß sie nur bedingt für den großtechnischen Einsatz geeignet sind. Problematisch ist hierbei vor allem, daß eine Kultivierung mit hoher Zellkonzentration im Bioreaktor nur eingeschränkt möglich ist, des weiteren führt eine vergleichsweise geringe Eduktkonzentration aufgrund von Inhibitionseffekten zum Abbruch der Reaktion. Die bekannten Verfahren sind weiterhin durch besondere Anforderungen an Kultivierungsbedingungen, niedrige Aktivität und geringe Spezifität der eingesetzten Mikroorganismen gekennzeichnet. Hierbei sind insbesondere die Labilität der bekannten Mikroorganismen gegenüber Temperatur- und pH-Wert-Schwankungen nachteilig. Desweiteren sind die bekannten Mikroorganismen schlecht oder gar nicht wiederverwertbar.

Die Aufgabe der vorliegenden Erfindung hat darin bestanden, ein Verfahren zur enzymatischen Umsetzung von Nitrilen und/oder Amiden zu den entsprechenden Amiden und/oder Carbonsäuren bereitzustellen. Hierbei war insbesondere der Einsatz des Verfahrens in großtechnischem Maßstab von Interesse. Dazu sollten die im Verfahren eingesetzten Mikroorganismen insbesondere eine Kultivierung in hoher Zellkonzentration sowie eine Tolerierung möglichst hoher Eduktkonzentration aufweisen. Eine hohe Aktivität sowie Spezifität der eingesetzten Mikroorganismen war ebenso wünschenswert wie der Einsatz der Mikroorganismen als Ganzes als auch in Form eines aus ihnen isolierten Enzymkomplexes.

Von besonderem Interesse war die selektive Umsetzung von Oligo- oder Polynitrilen zu entsprechenden Monoamiden bzw. zu Monocarbonsäuren. Wünschenswert war weiterhin eine gezielte Steuerung der Umsetzung durch einfache verfahrenstechnische Parameter, eine einfache Abtrennung der Mikroorganismen aus dem Reaktionssystem, hohe Stabilität der Mikroorganismen bei Lagerung sowie die Wiederverwendbarkeit nach Reaktionsende. Das Verfahren sollte weiterhin möglichst sowohl als kontinuierliches, als batch-Verfahren wie auch als fed-batch-Verfahren einsetzbar sein.

Es wurde gefunden, daß diese komplexen Aufgaben gelöst werden durch ein Verfahren bei dem man Mikroorganismen und/oder aus ihnen isolierte Nitrilhydratase-Amidase-Komplexe einsetzt, wobei die Mikroorganismen ausgewählt sind aus der Gruppe, die gebildet wird von Rhodococcus erythropolis DSM 13002, Rhodococcus erythropolis DSM 13475 und Rhodococcus erythropolis DSM 13476.

Es wurde gefunden, daß die erfindungsgemäßen Mikroorganismen sowie die aus ihnen isolierten Nitrilhydratase-Amidase-Komplexe in der Lage sind Verbindungen mit mindestens einer Nitrilfunktion und/oder mindestens einer Amidfunktion mit hoher Spezifität zu den entsprechenden Amiden und/oder Carbonsäuren umzusetzen. Ein Gegenstand der vorliegenden Erfindung betrifft daher ein Verfahren zur enzymatischen Umsetzung von Verbindungen mit mindestens einer Nitrilfunktion und/oder mindestens einer Amidfunktion mit mindestens einem Mikroorganismus und/oder mindestens einem aus diesem Mikroorganismus isolierten Nitrilhydratase-Amidase-Komplex, wobei der Mikroorganismus ausgewählt ist aus der Gruppe, die gebildet wird von Rhodococcus erythropolis DSM 13002, Rhodococcus erythropolis DSM 13475 und Rhodococcus erythropolis DSM 13476.

Die so erhaltenen Verbindungen tragen anstelle der Nitrilfunktion dann eine Amidfunktion bzw. eine Carbonsäurefunktion.

Besonders vorteilhaft am erfindungsgemäßen Verfahren ist, daß man die Verbindungen mit mindestens einer Nitrilfunktion sowohl zu Verbindungen mit der entsprechenden Amidfunktion als auch zu Verbindungen mit der entsprechenden Carbonsäurefunktion umsetzen kann und dies in Abhängigkeit der Reaktionsbedingungen gezielt steuern kann.

Verfahrensdurchführung

Das erfindungsgemäße Verfahren kann in einer Vielzahl von Varianten durchgeführt werden: Üblicherweise liegt das Substrat (Nitril und/oder Amid) in Reinform oder in Lösung vor und wird mit dem Mikroorganismus und/oder Nitrilhydratase-Amidase-Komplex des Mikroorganismus in Kontakt gebracht. Liegt das Substrat in Lösung vor, handelt es sich bevorzugt um eine wässerige Lösung. Das Substrat kann, falls erforderlich mittels eines Lösungsvermittlers in Lösung gebracht werden. Das Substrat kann, falls erforderlich in einem aprotischen Lösungsmittel gelöst werden. Beispielsweise seien Dimethylformamid und Dimethylsulfoxid genannt. Dabei kann das Substrat dem Anzuchtmedium in der Wachstumsphase der Mikroorganismen zugesetzt werden, es dient dann als weitere Kohlenstoff- und/oder Stickstoffquelle. Möglich ist ebenso die Anzucht der Mikroorganismen ohne Substratzusatz und Zugabe des Substrates nach Erreichen der stationären Wachstumsphase der Mikroorganismen. Weiterhin möglich ist die Anzucht der Mikroorganismen (mit oder ohne Substrat), Abtrennung vom Kulturmedium beispielsweise durch Filtration, Zentrifugation, Resuspension in einem geeigneten Medium und Zugabe des Substrates. Ebenso können die Mikroorganismen zur Isolierung des Nitrilhydratase-Amidase-Komplexes mit oder ohne Substratzusatz gezüchtet werden, nach Isolierung des Nitrilhydratase-Amidase-Komplexes erfolgt dann die Inkubation mit dem Substrat wie oben beschrieben erfolgen.

Die Wahl der Zugabeform des Substrates (in Reinform oder in Lösung) richet sich nach der Löslichkeit des Substrates. Bevorzugt wird das Substrat in Reinform zugegeben, da so eine unerwünschte Verdünnung des Reaktionsansatzes vermieden wird.

Bei einer kontinuierlichen Zudosage des Substrates hat es sich als vorteilhaft erwiesen im Reaktionsansatz die Konzentration an Substrat einzustellen, die im Aktivitätsmaximum des Enzymsystems liegt. Für Propionitril liegt diese Konzentration zwischen 100 und 300 mM.

Für die enzymatische Umsetzung werden die Mikroorganismen in Form ganzer Zellen üblicherweise entsprechend einer Biotrockenmassekonzentration von 0,2 bis 50 g/l, insbesondere 1 bis 10 g/l eingesetzt. Zur Isolierung der Nitrilhydratase-Amidase Komplexe werden üblicherweise 20 bis 100, insbesondere 30 bis 80 g/l Biotrockenmasse eingesetzt. Die Biotrockenmasse wird durch Wiegen von 10 ml Zellsuspension nach Trocknen bei 100°C für 4 h bestimmt. Sowohl die Mikroorganismen als auch die aus ihnen isolierten Nitrilhydratase-Amidase-Komplexe können auch in Form von Lyophilisaten eingesetzt werden. Insbesondere führt die Lyophilisation zu einer Verbesserung der Lagerstabilität. Die Mikroorganismen bzw. die daraus isolierten Nitrilhydratase-Amidase-Komplexe werden üblicherweise in einem geeigneten Reaktionspuffer resuspendiert. Als Reaktionspuffer eignen sich wässerige Salzlösungen bzw. Puffersysteme. Beispiele für geeignete Reaktionspuffer sind wässerige Hydrogenphosphat-, Dihydrogenphosphat-Lösungen und/oder Tris-Puffer Systeme, die üblicherweise eine pH-Wert von 6,5 bis 10,5, insbesondere von 7 bis 8, insbesondere von 7,2 aufweisen.

Die Umsetzung kann bei einer Temperatur von 10 bis 40°C, insbesondere von 15 bis 40°C, bevorzugt von 20 bis 38°C durchgeführt werden. Der Verlauf der Umsetzung kann durch Nachweis der Substrate und/oder Produkte im Reaktionsansatz, beispielsweise durch chromatographische Methoden (z.B. HPLC) verfolgt werden. Die Isolierung der Produkte aus dem Reaktionsansatz kann durch übliche Aufreinigungstechniken, wie beispielsweise Trocknung, Zentrifugation, Membranseparation, Vakuumkonzentration oder Kristallisation erfolgen. Einer Aufreinigung der Produkte kann gegebenenfalls eine Aktivkohlebehandlung und/oder eine Ionenaustauschchromatographie vorangestellt werden.

Die im erfindungsgemäßen Verfahren hergestellten Produkte lassen sich vorteilhaft aus der wäßrigen Reaktionslösung über Extraktion oder Kristallisation oder über Extraktion und Kristallisation gewinnen. Hierzu wird die wäßrige Reaktionslösung mit einer Säure wie einer Mineralsäure (z.B. HCl oder $H_2SO_4$) oder einer organischen Säure angesäuert vorteilhaft auf pH-Werte unter 2 und anschließend mit einem organischen Lösungsmittel extrahiert. Die Extraktion kann zur Erhöhung der Ausbeute mehrfach wiederholt werden. Als organische Lösungsmittel können prinzipiell alle Lösungsmittel verwendet werden, die mit Wasser gegebenenfalls nach Zugabe von Salzen eine Phasengrenze zeigen. Vorteilhafte Lösungsmittel sind Lösungsmittel wie Toluol, Benzol, Hexan, Methyltertiärbutylether oder Essigester.

Nach Einengen der organischen Phase können die Produkte in der Regel in guten chemischen Reinheiten, das heißt größer 90 % chemische Reinheit, gewonnen werden. Nach Extraktion kann die organische Phase mit dem Produkt aber auch nur zum Teil eingeengt werden und das Produkt auskristallisiert werden. Dazu wird die Lösung vorteilhaft auf eine Temperatur von 0 bis 10°C abgekühlt. Die Kristallisation kann auch direkt aus der organischen Lösung erfolgen. Das auskristallisierte Produkt kann nochmals im gleichen oder in einem anderen Lösungsmittel zur erneuten Kristallisation aufgenommen werden und nochmals kristallisiert werden. Durch die anschließende mindestens einmalige Kristallisation kann die Enantiomerenreinheit des Produktes je nach Lage des Eutektikum weiter gesteigert werden.

Die Produkte können jedoch auch direkt nach Ansäuern mit einer Säure auf einen pH-Wert vorteilhaft unter 2 aus der wäßrigen Reaktionslösung auskristallisiert werden. Vorteilhaft wird dazu die wäßrige Lösung unter Erwärmen eingeengt und in ihrem Volumen um 10 bis 90 %, bevorzugt 20 bis 80 %, besonders bevorzugt 30 bis 70 % reduziert. Vorzugsweise wird die Kristallisation unter Kühlung durchgeführt. Temperaturen zwischen 0 bis 10°C sind für die Kristallisation bevorzugt. Aus kostengründen ist die direkte Kristallisation aus der wäßrigen Lösung bevorzugt. Ebenfalls bevorzugt wird eine Aufarbeitung der Produkte über ein Extraktion und gegebenenfalls anschließender Kristallisation.

Bei diesen bevorzugten Aufarbeitungsarten läßt sich das Produkt des erfindungsgemäßen Verfahrens in Ausbeuten von 60 bis 100 %, bevorzugt von 80 bis 100 %, besonders bevorzugt von 90 bis 100 % bezogen auf das für die Reaktion eingesetzte Substrat isolieren. Das isoliert Produkt zeichnet sich durch eine hohe chemische Reinheit von > 90 %, bevorzugt > 95 %, besonders bevorzugt von > 98 % aus.

Die so gewonnenen Produkte eignen sich als Ausgangsmaterial für organischen Synthesen zur Herstellung von Pharmaka oder Agrochemikalien oder zur Racematspaltung.

Die vorliegende Erfindung umfaßt die Erkenntnis, daß die enzymatische Aktivität der Mikroorganismen und/oder der aus ihnen isolierten Nitrilhydratase-Amidase-Komplexe gezielt, je nach Wahl der Kultivierungsbedingungen und der Verfahrensparameter, insbesondere des pH-Wertes und der Reaktionstemperatur, gesteuert werden kann. Der Fachmann wählt in Abhängigkeit der gewünschten enzymatischen Umsetzung die Kultivierungsbedingungen, den Reaktionspuffer sowie die Reaktionsbedingungen. Von besonderer Bedeutung ist hierbei die Wahl des pH-Wertes des Reaktionspuffers: wird als Substrat eine Verbindung mit einer Nitrilfunktion eingesetzt und ist die Umsetzung zum Amid (Nitrilhydratase Reaktion) gewünscht, haben sich folgenden Verfahrensparameter als vorteilhaft erwiesen: pH 8 bis 10,5, insbesondere pH 9 bis 10. Wird als Substrat, eine Verbindung mit einer Nitrilfunktion, eingesetzt und die Umsetzung zur organischen Säure gewünscht (Nitrilhydratase-Amidase Reaktion), haben sich folgenden Verfahrensparameter als vorteilhaft erwiesen: pH 6 bis 10,5, insbesondere 7 bis 9, Wird als Substrat eine Verbindung mit einer Amidfunktion eingesetzt (Amidase Reaktion) sind folgenden Verfahrensparameter vorteilhaft pH 6 bis 9, insbesondere pH 7 bis 8.

Wird als Substrat eine Verbindung mit Nitril- und Amidfunktion eingesetzt und ist die selektive Umsetzung der Nitril bzw. der Amidfunktion erwünscht (entweder Nitrilhydratase- oder Amidase-Reaktion), kann dies der Fachmann durch gezielte Auswahl des pH-Wertes und der Temperatur wählen. Die vorliegende Erfindung schließt die Erkenntnis ein, daß das erfindungsgemäße Verfahren zum einen zum gezielten Abbau von Nitrilen und/oder Amiden wie auch zur Synthese von Amiden und/oder Carbonsäuren eingesetzt werden kann.

Verbindungen mit mindestens einer Nitrilfunktion

Verbindungen, die mindestens eine Nitrilfunktion (R-CN) tragen und die mit den erfindungsgemäßen Mikroorganismen und/oder den daraus isolierten Nitrilhydratase-Amidase-Komplexen umgesetzt werden können sind beispielsweise gesättigte aliphatische Nitrile, wie Acetonitril, Propionitril, Bernsteinsäurenitril, Adipinsäurenitril, ethylenisch ungesättigte aliphatische Nitrile, wie Acrylnitril und Methacrylonitril, aromatische Nitrilen wie Benzonitril und Phthalsäurenitril und heterocyclische Nitrile, wie Nicotinsäurenitril. Weiterhin geeignet sind Dinitrile, wie beispielsweise Phthalsäuredinitril, Bernsteinsäuredinitril, Adipinsäuredinitril sowie Polynitrile, wie z.B. Tricyanohexan. Besonders bevorzugt ist Propionitril. Die Nitrile können in einer Konzentration von 0,5 bis 200 g/l, besonders von 2 bis 150 g/l, insbesondere von 5,0 bis 60 g/l, eingesetzt werden. Prinzipiell geeignet sind Verbindungen mit einer unbegrenzten Anzahl an Nitrilfunktionen. Besonders geeignet sind Verbindungen mit 5 oder weniger Nitrilfunktionen. Besonders bevorzugt werden Verbindungen mit einer und/oder zwei Nitrilfunktionen eingesetzt.

Verbindungen mit mindestens einer Amidfunktion

Verbindungen, die mindestens eine Amidfunktion tragen und die mit den erfindungsgemäßen Mikroorganismen und/oder den daraus isolierten Nitrilhydratase-Amidase-Komplexen umgesetzt werden können sind beispielsweise gesättigte aliphatische Amide, ethylenisch ungesättigte aliphatische Amide, aromatische Amide und heterocyclische Amide. Beispielsweise seien genannt: Propionamid, Acetoamid, Succinoamid, Adipoamid, Acrylamid, Methacrylamid, Benzamid, Phthalsäureamid, Nicotinamid. Geeignete Diamide sind beispielsweise Phthalsäurediamid, Bernsteinsäurediamid und Adipinsäurediamid. Besonders geeignet sind Propioamid, Acetoamid und/oder Acrylamid. Die Amide können in

einer Konzentration von 0,5 bis 500 g/l, insbesondere von 5,0 bis 60 g/l, besonders bevorzugt von 7 bis 25 g/l eingesetzt werden. Prinzipiell geeignet sind Verbindungen mit einer unbegrenzten Anzahl an Amidfunktionen. Besonders geeignet sind Verbindungen mit 5 oder weniger Amidfunktionen. Besonders bevorzugt sind Verbindungen mit einer und/oder zwei Amidfunktionen.

Weiterhin eignen sich als Substrate Verbindungen, die sowohl mindestens eine Nitril- als auch eine Amidfunktion tragen.

In einer weiteren Ausführungsform des Verfahrens können die Mikroorganismen und/oder die daraus isolierten Nitrilhydratase-Amidase Komplexe immobilisiert werden. Überraschender weise wurde gefunden, daß durch die Immobilisierung nicht nur eine einfache Abtrennung aus der Reaktionslösung und eine Wiederverwendbarkeit der Mikroorganismen und/oder der aus ihnen isolierten Nitrilhydratase-Amidase-Komplexe erzielt werden kann, sondern daß die Immobilisierung sich stabilisierend auf die Enzymaktivität, insbesondere auf die Aktivität der Amidase, auswirkt. Geeignet sind bekannte Methoden der Immobilisierung, wie beispielsweise die Quervernetzung mit Glutaraldehyd oder die Bindung an Träger. Es hat sich als vorteilhaft erwiesen, die Immobilisierung bei 0 bis 10°C, insbesondere bei 4°C durchzuführen. Die Bindung an einen Träger erfolgt üblicherweise durch Adsorption, Ionenbindung oder kovalente Bindung. Beispielhaft ist der Einschluß in ein Alginatpolymer (Immobilisierung ganzer Zellen) zu nennen oder der Einschluß in ein kovalentes Netzwerk auf Basis von Acrylamid (kovalente Bindung).

In einer bevorzugten Ausführungsform der vorliegenden Erfindung werden die Mikroorganismen und/oder die daraus isolierten Nitrilhydratase-Amidase-Komplexe in Form von Alginatpolymeren immobilisiert. Diese Immobilisierung eignet sich insbesondere für die Mikroorganismen in Form ganzer Zellen. Die Immobilisierung in Form von Alginatpolymeren erfolgt üblicherweise durch Herstellen einer Alginatlösung, die 1:1 (Volumenanteile) mit einer Zellsuspension gemischt wird. Es hat sich als vorteilhaft erwiesen das Verhältnis der Konzentration der Zellsuspension zur Konzentration der Alginatlösung im Bereich von 20:1 bis 5:1, insbesondere von 15:1 bis 7,5:1 zu wählen. Besonders bevorzugt ist ein Verhältnis von 10:1. Das bedeutet man setzt beispielsweise eine 30 Gew.-%ige Zellsuspension zu einer 3 Gew.-% Alginatlösung zu. Werden anstelle der Mikroorganismen die aus ihnen isolierten Nitrilhydratase-Amidase-Komplexe immobilisiert, wird eine entsprechend geringere Konzentration eingesetzt, die sich für den Fachmann durch einfach Berechnung ergibt. Üblicherweise wird die Mischung von Zellsuspension und/oder Nitrilhydratase-Amidase-Komplex und Alginatlösung mittels Sprühvertropfung in die Vernetzerlösung gesprüht. Als Vernetzer eignet sich beispielsweise eine $CaCl_2$-Lösung. Dabei kommt es zum Austausch von einwertigen $Na^+$ Ionen des Alginats mit zweiwertigen $Ca^{2+}$Ionen der Vernetzerlösung, welche dann die Alginatstränge miteinander verbinden. Man erhält Alginatpolymere, deren Durchmesser in Abhängigkeit von den verwendeten Sprühköpfen eingestellt werden kann. Übliche Durchmesser liegen im Bereich von 0,5 bis 2 mm, insbesondere 0,8 und 1,0 mm. Als besonders vorteilhaft, insbesondere für die Stabilisierung der Enzymaktivität hat sich erwiesen, die Alginatimmobilisierung so durchzuführen, daß die Alginatperlen einen Durchmesser von 0,8 bis 1,0 mm aufweisen.

In einer weiteren Ausführungsform erfolgt die Immobilisierung durch Einschluß in ein kovalentes Netzwerk auf Basis von Acrylamid. Dabei hat es sich als vorteilhaft erwiesen die Acrylamid-, Bisacrylamid-, TEMED sowie Ammoniumpersulfatlösungen bei Raumtemperatur vor zu polymerisieren, und danach die Zellsuspension und/oder die isolierte Nitrilhydratase-Amidase-Komplexe, vorzugsweise bei 4°C, zuzugeben. Das Verhältnis von Acrylamid zu Bisacrylamid liegt üblicherweise im Bereich von 40:1 bis 2:1, bevorzugt bei 30:1 bis 5:1, insbesondere 10:1 bis 8:1. Die vorliegenden Erfindung schließt die Erkenntnis mit ein, daß durch eine Erniedrigung des Acrylamidanteils eine Erhöhung der Enzymaktivitäten, insbesondere der Nitrilhydrataseaktivität, erzielt werden kann.

Das erfindungsgemäße Verfahren kann sowohl kontinuierlich als auch in absatzweiser Prozeßführung (batch-Verfahren) sowie in absatzweiser Prozeßführung mit kontinuierlicher und/oder diskontinuierlicher Substratzugabe (fed-batch sowie repeatedfed-batch) durchgeführt werden. Das Verfahren insbesondere unter Einsatz von immobilisierten Mikroorganismen und/oder immobilisierten Nitrilhydratase-Amidase-Komplexen kann sowohl im Rührreaktor als auch im Festbett durchgeführt werden.

Als besonders vorteilhaft zur Erzielung einer hohen Produktkonzentration bei gleichzeitiger Vermeidung einer Inaktivierung hat es sich erwiesen das erfindungsgemäße Verfahren in absatzweiser Prozeßführung mit kontinuierlicher Substratzugabe durchzuführen. Besonders bevorzugt ist hier der Einsatz von immobilisierten Mikroorganismen und/oder immobilisierten Nitrilhydratase-Amidase-Komplexen.

Die vorliegende Erfindung schließt die Erkenntnis mit ein, daß je nach ausgewählter Prozeßführung und nach ausgewählten Prozeßparametern die Nitrilhydratase- und/oder Amidase-Aktivität gezielt gesteuert werden kann. Die folgenden Tabellen fassen die bei verschiedenen Prozeßführungen erhaltenen Parameter für die Nitrilhydratase- bzw. Amidase-Reaktion zusammen.

| Nitrilhydratase | Aktivität (apparent) [U/g BTM] | Halbwertzeit der Enzymaktivität [h] | Produkt pro Biomasse [mol/g BTM] | BTM [g/l] | Reaktionszeit [h] | Reaktionsvolumen [m] |
|---|---|---|---|---|---|---|
| Prozeßführung | | | | | | |
| absatzweise | 9000 | nicht bestimmt | nicht bestimmt | 0,77 | 1 | 25 |
| absatzweise, wiederholter Einsatz (Immobilisat) | 5814 | 6,94 | nicht bestimmt | 1,55 | 8 | 25 |
| absatzweise, gepulste Substratzugebe (Immobilisat) | 9500 | 10,00 | 0,01 | 2,94 | 2 | 50 |
| absatzweise, Fed-batch | 75 | 46,00 | 0,75 | 4,00 | 366 | 500 |
| absatzweise, Fed-batch (Immobilisat | 67 | nicht bestimmt nicht bestimmt | 0,56 | 2,70 | 450 | 250 |
| kontinuierlicher Rührreaktor (Immobilisat) | 650 | 57,00 | 1,69 | 1,07 | 216 | 100 |
| Festbett/ Rohrreaktor (Immobilisat) | 92 | 138,00 | 2,33 | 3,00 | 400 | 20 |
| Amidase | spezifische Aktivität [U/g BTM] | Halbwertzeit der Enzymaktivität [h] | Produkt pro Biomasse [mol/g BTM] | BTM [g/l] | Reaktionszeit [h] | Reaktionsvolumen [m] |
| Prozeßführung | | | | | | |
| absatzweise | 300,0 | nicht bestimmt | nicht bestimmt | 0,77 | 1 | 25 |
| absatzweise, wiederholter Einsatz (Immobilisat) | 283,0 | nicht feststellbar | nicht bestimmt | 1,55 | 8 | 25 |
| absatzweise, gepulste Substratzugebe (Immobilisat) | 341,0 | nicht feststellbar | 0,01 | 2,94 | 2 | 50 |
| absatzweise, Fed-batch | 75,0 | 57,00 | 0,75 | 4,00 | 366 | 500 |
| absatzweise, Fed-batch (Immobilisat | 67,0 | nicht bestimmt | 0,56 | 2,70 | 450 | 250 |

(fortgesetzt)

| Prozeßführung | | | | | | |
|---|---|---|---|---|---|---|
| kontinuierlicher Rührreaktor (Immobilisat) | 40,0 | 111,00 | 1,80 | 1,07 | 1311 | 150 |
| Festbett/ Rohrreaktor (Immobilisat) | 6,1 | nicht feststellbar | 0,40 | 0,66 | 1050 | 25 |
| Aktivität (apparent) = beobachtbare spezifische Reaktionsrate;<br>Produkt pro Biomasse = Summe des entstandenen Produktes über die Reaktionszeit bezogen auf die eingesetzte Biomasse<br>Aktivität (apparent) = beobachtbare spezifische Reaktionsrate;<br>Produkt pro Biomasse = Summe des entstandenen Produktes über die Reaktionszeit bezogen auf die eingesetzte Biomasse;<br>nicht feststellbar = keine Inaktivierung über die Reaktionszeit | | | | | | |

Besonders bevorzugt ist die absatzweise Prozeßführung, da sie sowohl hinsichtlich der Nitrilhydratase als auch der Amidase-Aktivität die höchste spezifische Aktivität erreicht.

Insbesondere bevorzugt, u.a. hinsichtlich einer maximal möglichen Produktkonzentration, ist die absatzweise Prozeßführung mit diskontinuierlicher (= gepulster) Substratzugabe.

Zur Erreichung einer möglichst hohen Produktkonzentration, bezogen auf die eingesetzte Biomasse, hat sich der Einsatz eines kontinuierlichen Rohrreaktors als vorteilhaft erwiesen.

In einer bevorzugten Ausführungsform des Verfahrens, der Reaktion im kontinuierlichen Rührreaktor, können hohe spezifische Aktivitäten für beide Enzyme bei zufriedenstellenden Produktmengen, bezogen auf die Biotrockenmasse, erhalten werden.

Die vorliegende Erfindung umfaßt die Erkenntnis, daß das erfindungsgemäße Verfahren auch mehrstufig durchgeführt werden kann. Hierzu eignet sich beispielsweise als erste Stufe die Durchführung im kontinuierlichen Rührreaktor mit anschließender Stufe im kontinuierlichen Festbettreaktor.

Der Fachmann kann in Kenntnis der einzelnen Parameter der Prozeßführungen die verschiedenen Stufen je nach gewünschter Umsetzung auswählen.

Gegenstand der vorliegenden Erfindung sind weiterhin der Stamm Rhodococcus erythropolis DSM 13002, der Stamm Rhodococcus erythropolis DSM 13475 sowie der Stamm Rhodococcus erythropolis DSM 13476 sowie jeweils Mutanten mit gleichen Eigenschaften. Als Mutanten mit gleichen Eigenschaften sind solche Mikroorganismen zu verstehen, die taxonomisch Rhodococcus erythropolis zu zuordnen sind und bei einer Umsetzung mit Propionitril mindestens 80 % der spezifischen Aktivität der erfindungsgemäßen Stämme aufweisen.

Diese Stämme wurden hinterlegt in der Deutschen Sammlung von Mikroorganismen und Zellkulturen GmbH (DSMZ Braunschweig) unter den Nummern DSM 13002, DSM 13475 und DSM 13476.

Alle 3 Stämme weisen die folgenden chemotaxonomischen Merkmale auf

- diagnostische Aminosäure des Peptidoglycan: meso-Diaminopimelinsäure
- Zucker aus Ganzzell-Hydrolysaten: Arabinose und Galaktose
- Mykolsäuren: Rhodococcus Mycolsäuren mit einer Kettenlänge von $C_{32}$-$C_{46}$
- Fettsäuremuster: Unverzweigte gesättigte und ungesättigte Fettsäuren plus Tuberculostearinsäure
- Menachinone: MK-8 ($H_2$)

Weiterhin sind alle 3 Stämme grampositiv, aerob und mesophil und bilden hellgelbe bis beige Kolonien.

Zusätzlich wurde für den Stamm Rhodococcus erythropolis DSM 13002 eine Komplettsequenzierung der 16S-rDNA durchgeführt, die eine > 99,5%ige Übereinstimmung mit den Sequenzen der spezifischen Regionen von Rhodococcus erythropolis ergab.

Anzuchtbedingungen

Die Mikroorganismen werden üblicherweise auf Agarplatten in LB-Komplexmedium gehalten wie in Beispiel 1 beschrieben. Die Anzucht in Kulturmedium erfolgt üblicherweise in Flüssigmedium in Schüttelkolben mit Schikanen und/ oder verschiedenen Rührreaktoren sowie in größeren Mengen in Biomassereaktoren. Die Flüssigmedien können als

Kohlenstoff- und Stickstoffquelle ausschließlich ein Nitril oder Mischungen verschiedener Nitrile, wie beispielsweise Propionitril, Acetonitril, Isobutyronitril, Methacrylonitril, Benzonitril, Lactonitril, Tricyanhexan, Hydroxypivalaldehydcyanhydrin enthalten, welche in einer Konzentration von 0,5 bis 10 g/l, vorzugsweise 2 bis 7 g/l, insbesondere von 4 bis 6 g/l eingesetzt werden. Bevorzugt ist die Kultivierung der Mikroorganismen in einem Kulturmedium, welches als Kohlenstoffquelle Glucose und als Stickstoffquelle Ammoniumsulfat und/oder Ammoniumnitrat enthält. Üblicherweise wird als Kohlenstoffquelle Glucose in einer Menge von 0,5 bis 20 g/l, insbesondere 1 bis 5 g/l eingesetzt. Vorteilhafterweise wird als Kohlenstoffquelle Glucose in einer Konzentration von 2 g/l eingesetzt. Als weitere oder alternative Kohlenstoffquellen sind Succinat, Acetat und/oder Citrat geeignet. Je nach gewünschter Aktivität (Nitrilhydratase und/oder Amidase) kann der Fachmann die Kultivierungsbedingungen wählen. Die vorliegende Erfindung umfaßt die Erkenntnis, daß durch Zusatz von Glucose insbesondere die Nitrilhydratase-Aktivität erhöht werden kann, wohingegen die Amidase-Aktivität durch Kultivierung in glucosefreiem Medium erhöht werden kann.

In einer Ausführungsform der Erfindung können die Nitrilhydratase und die Amidase-Aktivität selektiv durch die Wahl der Glucosekonzentration im Medium gesteuert werden.

Als Stickstoffquelle wird üblicherweise Ammoniumsulfat in einer Menge von 0,5 bis 10 g/l insbesondere 1 bis 5 g/l eingesetzt. Die vorliegende Erfindung umfaßt die Erkenntnis, daß der Zusatz von Ammonium, insbesondere in Form von Ammoniumsulfat die Aktivität des Nitrilhydratase-Amidase-Komplexes deutlich erhöht, insbesondere die Aktivität der Amidase wird erhöht. In einer Ausführungsform kann das Kulturmedium ein Nitril als Kohlenstoff- und Stickstoffquelle sowie zusätzlich eine weitere Kohlenstoff und eine weitere Stickstoffquelle enthalten. Das Kulturmedium enthält üblicherweise eine organische Nährstoffquelle, wie Hefeextrakt, Malzextrakt, Pepton und/oder Fleischextrakt. Zu diesem Medium kann eine anorganische Nährstoffquelle, die beispielsweise Phosphate, Natrium, Kalium, Eisen, Magnesium, Mangan und/oder Zink enthält, gegeben werden. Gegebenenfalls kann dem Medium eine Vitaminlösung zugesetzt werden. Die vorliegende Erfindung umfaßt weiterhin die Erkenntnis, daß der Zusatz von Eisen, insbesondere in Form von Eisen(III)sulfat zu einer Aktivitätssteigerung der Nitrilhydratase-Aktivität führt, wohingegen bei Kultivierung in Abwesenheit von Eisen die Amidase-Reaktion vollständig unterdrückt werden kann. Die in Beispiel 2 als Medium 1 und Medium 2 bezeichneten Flüssigmedien stellen für die Anzucht geeignete Flüssigmedien dar. Zur Erzielung einer hohen Nitrilhydratase-Amidase-Aktivität, hat es sich als vorteilhaft erwiesen, die Mikroorganismen in Medium 2 anzuzüchten.

Der pH-Wert des Kulturmediums liegt in der Regel zwischen pH 5 bis pH 9, insbesondere zwischen pH 6 bis pH 8. Die Anzuchttemperatur liegt üblicherweise zwischen 10 und 40°C, insbesondere zwischen 20 und 35°C, bevorzugt zwischen 27 und 32°C. Standardbedingungen zur Anzucht sind pH 7,2 und 30°C. Vorteilhafterweise wird mittels Rührer oder Sauerstoffbegasung eine möglichst hohe Sauerstoffsättigung der Kulturlösung erreicht, um das Wachstum der strikt aeroben Mikroorganismen nicht zu limitieren. Unter diesen Bedingungen ist eine aerobe Kultivierung in der Regel 2 bis 5 Tage möglich. Bei der Anzucht im Biomassereaktor hat es sich als vorteilhaft erwiesen im Laufe der Kultivierung bei Erreichen der stationären Wachstumsphase Hefeextrakt und/oder Ammoniumsulfat zuzugeben, diese erneute Zudosierung kann mehrfach erfolgen. Unter den genannten Bedingungen ist es möglich, die Mikroorganismen bis zu einer Konzentration von 1 bis 5 g Biotrockenmasse (BTM)/l zu kultivieren. Nach Erreichen der stationären Wachtumsphase werden die Mikroorganismen üblicherweise mittels Zentrifugation abgetrennt und können dann direkt für das Verfahren eingesetzt werden (= Biomasse), als Ausgangsmaterial für eine Immobilisierung eingesetzt werden oder zur Isolierung des Nitrilhydratase-Amidase-Komplexes eingesetzt werden. Je nach gewünschter Aktivität (Nitrilhydratase und/oder Amidase) kann der Fachmann die Kultivierungsbedingungen wählen. Die Anzucht der Mikroorganismen kann mit oder ohne den Zusatz eines Induktors durchgeführt werden.

Wird dem Anzuchtmedium ein Induktor zugegeben, so wird dieser üblicherweise in einer Konzentration von 0,2 bis 7,5 g/l zugesetzt. Bei den Induktoren kann es sich um Nitrile handeln, die als Kohlenstoff- und Stickstoffquelle dienen, geeignet sind jedoch auch andere Verbindungen wie Amide und Lactame. Als Induktoren geeignet sind beispielsweise Propionitril, Butyronitril, Isovaleronitril, Isobutyronitril, Propionamid, Acrylamid, Butyramid, Crotonamid, Methacrylamid, Isobutyramid und Harnstoff, Besonders geeignet als Induktoren sind Crotonsäurenitril, Crotonamid, Methacrylamid und Lactame. Als Lactame bevorzugt sind intramolekulare Amide von γ-Aminosäuren mit 4 bis 6 C-Atomen, insbesondere ε-Caprolactam, γ-Butyrolactam (= Pyrrolidon) und δ-Valerolactam (= 2-Piperidon). Insbesondere geeignet ist ε-Caprolactam in einer Konzentration von 0,5 bis 5,0 g/l.

Isolierung des Nitrilhydratase-Amidase-Komplexes

Ein weiterer Gegenstand der vorliegenden Erfindung betrifft den Nitrilhydratase-Amidase-Komplex aus einem Mikroorganismus, der ausgewählt ist aus der Gruppe, die gebildet wird von Rhodococcus erythropolis DSM 13002, Rhodococcus erythropolis DSM 13475 und Rhodococcus erythropolis DSM 13476. Die Isolierung des Nitrilhydratase-Amidase-Komplexes aus den beanspruchten Mikroorganismen kann dabei nach Verfahren des Standes der Technik durchgeführt werden. Dabei werden die Mikroorganismen zunächst bis zur gewünschten Zelldichte gezüchtet und durch Zentrifugation vom Anzuchtmedium getrennt. Der Zellaufschluß kann beispielsweise mittels enzymatischer Behandlung

(Lysozym) und anschließender mechanischer Behandlung (French Press, Ultraschallaufschluß) durchgeführt werden. Alternativ ist der Zellaufschluß direkt ohne vorherige Lysozymbehandlung in einer Glasmühle unter Stickstoff-Kühlung möglich. Die Abtrennung der Zellmembrane aus dem Zellaufschluß erfolgt üblicherweise durch Zentrifugation. Vorteilhafterweise werden alle Schritte der Isolierung des Nitrilhydratase-Amidase-Komplexes bei 4°C durchgeführt. Der Überstand aus dieser Zentrifugation enthält den Nitrilhydratase-Amidase-Komplex, der direkt eingesetzt werden kann oder gegebenenfalls durch weitere Verfahrensschritte aufgereinigt werden kann. Als weitere Verfahrensschritte seien exemplarisch genannt: Ultrazentrifugation, Aussalzen, Fällung mit organischen Lösungsmitteln, sowie Reinigung mittels Adsorptionschromatographie, Ionenaustauschchromatographie, hydrophobe Interaktionschromatographie (HIC) und/oder Gelpermeationschromatographie.

Beispiel 1: Stammhaltung auf Agarplatte

| Mediumkomponente | Konzentration |
|---|---|
| Bacto Trypton, Difco 0123-15-5 | 10 g/l |
| Hefeextrakt, Gibco BRL, UK 20047-056 | 5 g/l |
| NaCl | 10 g/l |
| Agar | 15 g/l |

Die Substanzen wurden in 970 ml VE-Wasser gelöst, mit 1 M NaOH auf pH 7,2 eingestellt und dann bis 1 l Endvolumen aufgefüllt.

Die Lösungen wurden bei 121°C und 1 atü Druck für 21 min sterilisiert und unter der Sterilbank in kommerziell erhältliche sterile Plastikpetrischalen gegossen. Nach dem Abkühlen wurden die Platten steril verpackt und bei 4°C gelagert.

Die Reinkultur der Mikroorganismen DSM 13002, DSM 13475 und DSM 13476 wurde regelmäßig alle vier Wochen auf LB-Agarplatten überimpft, für zwei Tage bei 30°C im Brutraum inkubiert und anschließend bei 4°C im Kühlraum gelagert.

Beispiel 2: Medien zur Zellanzucht in Flüssigkultur

| Medium 1 | | Medium 2 | |
|---|---|---|---|
| Separat autoklavieren | | Separat autoklavieren | |
| $K_2HPO_4$ | 0,5 g/l | $Na_2HPO_4$ | 0,5 g/l |
| $KH_2PO_4$ | 0,5 g/l | $KH_2PO_4$ | 0,5 g/l |
| Separat autoklavieren | | Sterilfiltriert | |
| $(NH_4)_2SO_4$ | 2 g/l | Citronensäure 0,2 | g/l |
| $MgSO_4$ x 7 $H_2O$ | 0,5 g/l | Fe(III)$SO_4$ x 7 $H_2O$ | 0,2 g/l |
| $CoCl_2$ | 10 mg/l | Separat autoklavieren | |
| Caprolactam | 0,5 g/l | $(NH_4)_2SO_4$ | 2 g/l |
| Hefeextrakt | 3 g/l | $MgSO_4$ x 7 $H_2O$ | 1,1 g/l |
| SL5 (s. unten) | 2 ml/l | $CaCl_2$ x 2 $H_2O$ | 0,05 g/l |
| | | Hefeextrakt | 2 g/l |
| | | SL6 (s. unten) | 1 ml/l |
| Separat autoklavieren | | Separat autoklavieren | |
| Glucose | 2 g/l | Glucose | 2 g/l |

Alle Komponenten außer der Glucoselösung wurden vor der Sterilisation mit NaOH auf pH 7,2 eingestellt.

Komponenten der Spurenelementlösungen SL5 und SL6

| SL5 | | SL6 | |
|---|---|---|---|
| EDTA | 2,5 g/l | EDTA | 5 g/l |
| Fe(III)$SO_4$ x $H_2O$ | 1 g/l | $ZnSO_4$ x 7 $H_2O$ | 1,1 g/l |
| $ZnSO_4$ x 7 $H_2O$ | 0,05 g/l | $CuSO_4$ x 5 $H_2O$ | 0,04 g/l |
| $MnCl_2$ x 4 $H_2O$ | 0,015 g/l | $MnSO_4$ x 1 $H_2O$ | 6 g/l |
| $H_3BO_3$ | 0, 15 g/l | $H_3BO_3$ | 0,06 g/l |
| $CoCl_2$ x 6 $H_2O$ | 0,1 g/l | KJ | 0,01 g/l |
| $CuCl_2$ x 2 $H_2O$ | 0,005 g/l | | |
| $NiCl_2$ x 6 $H_2O$ | 0,01 g/l | | |
| $Na_2MoO_4$ x 2 $H_2O$ | 0,015 g/l | | |

Die Medien wurden in der obengenannten Reihenfolge angesetzt. Dazu wurden konzentrierte Lösungen hergestellt: 800 ml Salze (zusammen mit der durch Sterilfiltration zugegebenen Eisenlösung), 150 ml Puffer und 50 ml Glucose für insgesamt 1 Liter Lösung, so daß sich nach dem sterilen Zusammengießen dieser Lösungen die oben beschriebenen Konzentrationen ergaben.

Die Eisensalzlösungen des Mediums 2 wurden mit gleichen Gewichtsanteilen (w/w) an Citronensäure hergestellt, wobei die maximale Löslichkeit bei 2 g/l Eisensalze lag. Nach dem Einstellen des pH-Wertes auf pH 7,2 wurden die Lösungen über eine Sterilfiltration mit sterilen 0,2 µm Filtern (Gelman Science, Michigan, USA) zu den restlichen autoklavierten Mediumkomponenten zugegeben.

Die Anzucht mit dem Medium 2 ergab eine deutlich höhere spezifische Aktivität der Nitrilhydratase von 60 000 U/g BTM und der Amidase von 400 U/g BTM gegenüber den spezifischen Aktivitäten aus der Anzucht mit Medium 1.

Beispiel 3: Biomasseproduktion im Schüttelkolben

Für die Biomasseproduktion wurden im Erlenmeyerkolben mit Schikanen (Schott, Mainz, D) mit 250, 500, 1000 oder 5000 ml Füllvolumen verwendet.

Diese wurden mit 20 % des maximalen Füllvolumens des Kolbens des jeweilig verwendeten Mediums ohne Glucose und, bei Medium 1, auch ohne Phosphatpuffer befüllt, bei 121°C und 1 atü Druck für 21 min sterilisiert und dann unter einer sterilen Werkbank Variolab (Waldner, Wangen, D) mit den übrigen separat sterilisierten Mediumskomponenten befüllt. Die Kolben wurden nach dem Beimpfen bei 30°C auf einer Rotationsschüttelmaschine (New Brunswick Scientific, Edison, USA) bei 120 Upm für 48 h inkubiert.

Für die zahlreichen Versuche zur Mediumsoptimierung, die Aktivitätstests und die Biofeuchtmassebestimmung wurden die Zellen nach Ende der Kultivierung über Zentrifugation für 20 min bei 5000 Upm und 4°C in einem Rotor JA-10 mit einer Ultrazentrifuge (Beckmann J2-21 M/E) abgeerntet.

Für die Verwendung der Kultur als Animpfkultur (Inoculum) wurden die Zellsuspensionen steril zu den jeweiligen Medien in den Bioreaktoren zugegeben.

Beispiel 4: Biomasseproduktion im 100 l Bioreaktor

Zur Produktion einer größeren Menge einheitlicher Zellmasse wurde eine Kultivierung im 60 l Maßstab durchgeführt. Hierzu wurde ein Stahlbioreaktor (Typ 9230 FLAW IL, Bioengineering, Wald, CH) verwendet.

Zur Vorbereitung der Kultivierung wurden die $pO_2$- und die pH-Meßeinheit kalibriert. Die Waage, auf welcher der Bioreaktor stand, wurde tariert. Dann wurde der gesamte Bioreaktor zusammen mit 57 l vorgelegtem Phosphatpuffer (s. Beispiel 2 Medium 1) für 21 min bei 121°C und 2 bar sterilisiert. Zur pH-Regelung wurden je 3 l 10 % (w/v) NaOH- und 10 % (w/v) Phosphorsäurelösungen hergestellt und in den Vorratsgefäßen sterilisiert. Nach dem Abkühlen wurde die 500 ml konzentrierte Glucoselösung, 2,5 l konzentrierte Salz- und Hefeextraktlösung, Antischaum (Ucolub N115, Fragol Schmiermittelindustrie GmbH, Mühlheim/Ruhr, D) und zuletzt das Inoculum steril dem Reaktor zugegeben. Die

Probenentnahme erfolgte über ein dampfsterilisierbares Druckventil. Der Inhalt des Bioreaktors wurde nach der Anzucht durch einen Hahn am Boden des Bioreaktors abgelassen und in die Zellabtrennung überführt.

Für die Zellanzucht wurden die in der folgenden Tabelle dargestellten Bedingungen und Parameter eingestellt:

Anzuchtbedingungen für die Kultivierung im 100-1-Bioreaktor Typ 9230 FLAW IL.

| Parameter | | |
|---|---|---|
| | Belüftungsrate | 10 Nl/min |
| | Drehzahl | 150 Upm |
| | Temperatur | 30°C |
| | $pH_{soll}$ $\Delta pH$ | 7,2 0,5 |

| | |
|---|---|
| Einstellung des pH mit | 10 % (w/v) NaOH<br>(steril in drucküberlagertem Vorratsgefäß)<br>10 % (v/v) $H_3PO_4$<br>(steril in drucküberlagertem Vorratsgefäß) |

Es wurde eine Anzucht in drei Stufen durchgeführt. Die Vorkultur 1 wurde in fünf separaten 500 ml Erlenmeyerkolben mit Schikanen (Medium 1) von einer Agarplatte (s. Beispiel 1) mit einer Impföse in ein Volumen von 50 ml angeimpft und 48 h bei 30°C auf einem Schüttler inkubiert. Mit diesen Vorkulturen wurden dann jeweils fünf sterile 5000 ml Erlenmeyerkolben mit einem Inhalt von je 1 l (Medium 1) beimpft. Vor der Zugabe des Inokulums zur Kulturbrühe im Bioreaktor wurde eine Nullprobe aus dem Reaktor genommen und dann die 5 l Inoculum in den Reaktor eingefüllt, so daß insgesamt 64,6 l Kulturflüssigkeit im Reaktor vorlagen.

Nach dem Erreichen der stationären Phase nach 27,5 h wurden 1 Liter steriles Hefeextrakt/Ammoniumsulfat zugegeben, mit einer Konzentration von 173 g/l Hefeextrakt und 115 g/l Ammoniumsulfat. Zu diesem Zeitpunkt waren durch Probennahmen 7,9 l aus dem Bioreaktor entnommen worden. Nach 38,5 h Prozeßzeit wurden 5 l Konzentrat zugegeben, bestehend aus: 90,3 g/l Hefeextrakt und 60,2 g/l Ammoniumsulfat.

Über die Kultivierungszeit wurden in regelmäßigen Abständen sterile Proben entnommen und davon die optische Dichte und die Biotrockenmassekonzentration der pH-Wert und zusätzlich der Glucose- und Ammoniumwert bestimmt.

Des weiteren wurde über eine Meßsonde der Sauerstoffpartialdurck $pO_2$ des Mediums im Bioreaktor gemessen. Die Abgasanalyse lieferte kontinuierlich die Meßwerte für den prozentualen Volumengehalt an Sauerstoff $YO_2$ und Kohlendioxid $YCO_2$ in der Fermenterabluft. Diese wurden bei jeder Probennahme abgelesen und protokolliert.

Nach 54 h wurde die Kultivierung nach dem Erreichen der stationären Wachstumsphase der Zellen beendet. Der Inhalt des Bioreaktors wurde abgelassen und die Biofeuchtmasse daraus mit einer kontinuierlichen Cepa-Schnellzentrifuge (Typ GLE, Firma Carl Padberg, Lahr, D) gewonnen.

Zentrifugationsbedingungen:

| | |
|---|---|
| Trommeldurchmesser: | 44 mm; |
| Drehzahl: | 33750 Upm; |
| Durchflußrate: | 250 ml/min |

Die so erhaltene Biofeuchtmasse wurde zu je 100 g portioniert und bei 4°C zur sofortigen Verwendung gelagert bzw. bei -20°C eingefroren.

Beispiel 5: Aktivitätsbestimmung mit freien, ruhenden Zellen

Die aus der Kultivierung (Beispiel 4) erhaltene Biomasse wurde für die Messung der Nitrilhydratase und der Amidase Aktivitäten verwendet. Für diesen Aktivitätstest wurden Biotrockenmassekonzentrationen von 0,2 bis 0,7 g/l eingesetzt.

Beispiel 5a): Nitrilhydratase-Aktivität

Der Standardaktivitätstest mit freien, ruhenden Zellen wurde wie folgt durchgeführt:

In einem 50 ml Glasreaktor (EHS, Bovenden, D) wurden zu 25 ml Reaktionspuffer I eine Spatelspitze Zellmasse gegeben und diese durch Rühren mit einem Rührkern auf einem Magnetrührer suspendiert. Der Reaktor wurde über

einen beheizbaren Mantel temperiert, wobei die Temperatur mit einem Wasserbad konstant auf 26°C eingestellt war. Die Reaktion wurde durch Zugabe von 169 µl reinem Propionitril gestartet. Die Anfangskonzentration von Propionitril im Aktivitätstest betrug somit 100 mM. Nach definierten Zeitpunkten von 0, 1, 3, 5, 15, 30 und 60 Minuten wurde mit einer Spritze über einen 0,2 µm Filter (Gelman Science, Michigan, USA), Durchmesser 25 mm, jeweils 1 ml Probe gezogen und durch die dabei erfolgte Abtrennung der Zellen die Reaktion abgestoppt. Die Konzentrationen von Substrat und Produkt wurde mittels HPLC bestimmt. Von der restlichen verbliebenen Zellsuspension im Reaktor nach Beendigung des Versuches wurde die Biotrockenmasse bestimmt.

Für die Einzelbestimmung der Aktivität der Nitrilhydratase wurden Proben nach 0, 1, 3, 5 und 10 min genommen.

Beispiel 5b): Amidase-Aktivität

Experimente zur Einzelbestimmung der Aktivität der Amidase wurden ebenfalls wie in Beispiel 5a) beschrieben durchgeführt, jedoch mit der Änderung, daß als Substrat 312 µl einer 8 M Stammlösung von Propionamid in VE-Wasser eingesetzt wurden. Es wurde entsprechend weniger Volumen an Zellsuspension eingesetzt, um nach der Zugabe von Substrat ein Gesamtvolumen von 25 ml im Reaktor zu erhalten. Die Probennahme erfolgte nach 0, 1, 15, 30, 45 und 60 min.

Aktivitätsbestimmungen mit immobilisierten Zellen

Für die Bestimmung der Aktivitäten von immobilisierten Zellen wurden in einem mit 5 ml Reaktionspuffer 3 gefüllten 25 ml Standzylinder volumetrisch 2 ml des abgetropften Immobilisats eingemessen und mit Reaktionspuffer 3 auf 25 ml aufgefüllt. Die Reaktion erfolgte wie im Standardtest mit freien Zellen, allerdings erfolgte die Probennahme durch Abpipettieren von 1 ml der Suspension inclusive den Immobilisatperlen und anschließendem filtrieren. Dadurch wurde ein Anreichern des Immobilisates in der Reaktionslösung verhindert.

Puffersysteme für Ruhezellumsätze

| System | |
|---|---|
| Reaktionspuffer 1 | |
| $Na_2HPO_4$ | 50 mM |
| $NaH_2PO_4$ | 50 mM |
| pH | 7,2 |
| Reaktionspuffer 2 | |
| TRIS/HCl | 50 mM |
| pH | 7,2 |
| Reaktionspuffer 3 Reaktionspuffer 2 mit 0,5 g/l $CaCl_2$ | |
| Reaktionspuffer 1 wurde durch Mischen der beiden 50 mM Stammlösungen bis zum Erreichen des gewünschten pH-Wertes hergestellt. Reaktionspuffer 2 und 3 wurde durch Titration mit NaOH auf den gewünschten pH-Wert eingestellt. | |

Auswertung der Standardaktivitätstests

Die Aktivitäten der Zellen wurden immer auf die eingesetzte Biotrockenmasse im Reaktor bezogen. Dazu wurde bei immobilisierten Zellen die Biotrockenmasse über die verwendete Biofeuchtmasse zur Herstellung des Immobilisates und das eingesetzte Volumen an Immobilisat abgeschätzt. Der Umrechungsfaktor (UF) von Biofeuchtmasse zu Biotrokkenmasse wurde zu UF = 4,5 bestimmt. Um die Aktivitäten der Enzyme zu bestimmen, wurden aus den Meßpunkten durch Anlegen von Tangenten und Berechnung der jeweiligen Steigung über eine lineare Regression die Anfangsreaktionsraten der jeweiligen Produktbildung bestimmt. Dies erfolgte wie in Fig. 1 dargestellt.

Die Aktivität wurde als spezifische Aktivität bezogen auf die im Ansatz verwendete Biotrockenmassekonzentration in U/g BTM angegeben. Die Berechnung erfolgte durch Messung der Entstehung an Produkt zum Zeitpunkt des Reaktionsstarts. Die spezifische Aktivität berechnet sich dann entsprechend der Gleichung

$$r = \frac{\text{Konzentration [mM]}}{\text{Zeit [min]}} \times 1000 \times \frac{1}{\text{BTM [g/l]}} = \left[\frac{U}{gBTM}\right]$$

| | |
|---|---|
| r = | spezifische Anfangsreaktionsrate [U/g BTM] |
| Konzentration/Zeit = | Steigung der Tangenten an die Meßpunkte [mM/min] |
| BTM = | Biotrockenmassekonzentration im Ansatz [g/l] |

Beispiel 6: Bestimmung des Temperaturoptimums der Enzymreaktionen

Die optimale Temperatur für die Reaktionen der Nitrilhydratase und der Amidase im Ganzzellsystem wurde wie folgt bestimmt. Die Bedingungen für die Reaktion waren die des Standardaktivitätstests (s. Beispiel 5) bei pH 7,2. Die eingesetzte Substratkonzentration für die Reaktion lag bei 100 mM Propionitril für die Nitrilhydratasereaktion bzw. 100 mM Propionamid für die Amidasereaktion unter Verwendung eines 50 ml Glasreaktors mit 25 ml Mediumvolumen. Die Zellen wurden vor Beginn des Versuches für 5 min bei der jeweiligen Temperatur vorinkubiert und dann die Reaktion gestartet. Die Anfangsreaktionsraten der jeweiligen Enzymreaktionen wurde bei der untersuchten Temperatur im Standardaktivitätstest ermittelt. Die erhaltenen spezifischen Reaktionsraten wurden gegen die Temperatur aufgetragen (siehe Fig. 2).

Beispiel 7: Bestimmung des pH-Optimums der Enzymreaktionen

Das pH-Optimum für die spezifische Aktivität der Nitrilhydratase als auch der Amidase wurde wie folgt bestimmt.

Die Bedingungen für die Reaktion waren die des Standardaktivitätstests (s. Beispiel 5) bei 26°C. Die eingesetzte Substratkonzentration für die Reaktion lag bei 100 mM Propionitril für die Nitrilhydratasereaktion bzw. 100 mM Propionamid für die Amidasereaktion unter Verwendung eines 50 ml Glasreaktors mit 25 ml Mediumvolumen. Die Zellen wurden vor Beginn des Versuches für 5 min bei dem jeweiligen pH-Wert vorinkubiert und dann die Reaktion gestartet (siehe Fig. 3).

Die spezifische Aktivität der Nitrilhydratase zeigte ein ausgeprägtes Optimum bei pH 9,5, war aber noch bei pH 10,5 aktiv. Bei pH 6 war sie inaktiv.

Die spezifische Aktivität der Amidase zeigte ein eher breites Optimum zwischen pH 7,2 und pH 8,5. Die Amidase war, anders als die Nitrilhydratase, auch noch bei pH 6 aktiv.

Beispiel 8: Immobilisierung von Rhodococcus erythropolis DSM 13002

Beispiel 8a) Immobilisierung der Zellen in Alginat

Herstellung der Lösungen für die Alginatimmobilisierung

| Komponente | Zusammensetzung |
|---|---|
| Alginatlösung | 3 % (w/v) in $H_2O$ |
| Zellsuspension | 30 % (w/v) in Reaktionspuffer 2 |
| Vernetzerlösung | Reaktionspuffer 2 mit 1,0 g/l $CaCl_2$ |
| Reaktionspuffer 3 | Reaktionspuffer 2 mit 0,5 g/l $CaCl_2$ |

Die Alginatlösung wurde 1:1 mit der Zellsuspension gemischt und auf Eis gelagert. Dann wurde die Suspension über eine Schlauchpumpe in einen Apparat zur Sprühvertropfung gepumpt.

Die entstandenen Alginatperlen mit einem Durchmesser von etwa 0,9 mm wurden 30 min in der Vernetzerlösung ausgehärtet, mit Reaktionspuffer 3 gewaschen und bis zur weiteren Verwendung bei 4°C gelagert.

Das Immobilisat bestand nach dem Aushärten aus nahezu sphärischen Partikeln, die eine sehr gute Stabilität gegenüber den durch die Rührung mit einem Magnetkern im Standardaktivitätstest auftretenden Abriebs- und Scherkräften zeigten. Die Partikel wurden nach der Immobilisierung ausgesiebt, wobei bei 90 % der erhaltenen Partikel Korngrößen zwischen 0,8 und 1 mm bestimmt wurden. Weitere 5 % waren zwischen 1 und 1,4 mm groß, alle übrigen

größer oder kleiner als die angegebenen Durchmesser.

Beispiel 8b) Immobilisierung der Zellen in Acrylamid

Herstellung der Lösungen für die Acrylamidimmobilisierung

| Nr. | Komponente | Zusammensetzung |
|---|---|---|
| 1 | Acrylamidstammlösung 1 | Rotiphorese Gel30 (Roth GmbH+Co., Karlsruhe, D), 30 % Acrylamid, 0,8 % Bisacrylamid |
| 2 | Acrylamidstammlösung 2 | Rotiphorese Gel19 (Roth GmbH+Co., Karlsruhe, D), 19 % Acrylamid, 1 % Bisacrylamid |
| 3 | Acrylamidstammlösung 3 | 0.0277 g Bisacrylamid in 3,52 ml $H_2O$ bid gelöst |
| 4 | TEMED | N,N,N',N'-Tetra-methyl-ethylendiaminlösung (Bio-RAD Lab. München, D) |
| 5 | APS-Lösung | 10 %ige (w/v) Ammoniumpersulfatlösung in $H_2O$ bid (immer frisch angesetzt) |
| 6 | Zellsuspension | 3 g Biofeuchtmasse mit 2 ml Reaktionspuffer 2 suspendiert |

Ansatz 1

| Nr. | Komponente | Volumen |
|---|---|---|
| 2 | Acrylamidstammlösung 2 | 5 ml |
| 4 | TEMED | 75 µl |
| 5 | APS-Lösung | 150 µl |
| 6 | Zellsuspension | 5 ml |

Die Lösungen Nr. 2, 4, 5 wurden in einer Glaspetrischale gemischt und 2 min bei Raumtemperatur vorpolymerisiert. Dann wurde die Zellsuspension (6) zugegeben und mit einem Spatel untergemischt, wobei sorgfältig eine Blasenbildung vermieden wurde. Zur Auspolymerisierung wurde die Petrischale auf einer vorgekühlten Metallplatte für ca. 15 min gelagert. Nach Aushärtung des Geles wurde dieses mit Reaktionspuffer 2 gewaschen und bis zur Weiterverwendung bei 4°C im Kühlschrank gelagert.

Ansatz 2

| Nr. | Komponente | Volumen |
|---|---|---|
| 1 | Acrylamidstammlösung 2 | 5 ml |
| 5 | APS-Lösung | 5 ml |
| 4 | TEMED | 100 µl |
| 6 | Zellsuspension | 250 µl |

Die Lösungen Nr. 1 und 5 wurden ebenfalls in einer Glaspetrischale gemischt und 2 min bei Raumtemperatur inkubiert. Dann wurden die Lösungen Nr. 6 und 4 zugegeben und mit einem Spatel untergemischt, wobei sorgfältig eine Blasenbildung vermieden wurde. Zur Auspolymerisierung wurde wie im Ansatz 1 verfahren. Nach Aushärtung des Geles wurde dieses mit Reaktionspuffer 2 gewaschen und bis zur Weiterverwendung bei 4°C im Kühlschrank gelagert.

Ansatz 3

| Nr. | Komponente | Volumen |
|---|---|---|
| 2 | Acrylamidstammlösung 2 | 1,48 ml |
| 3 | Acrylamidstammlösung 3 | 3,52 ml |

(fortgesetzt)

| Nr. | Komponente | Volumen |
|---|---|---|
| 4 | TEMED | 250,00 µl |
| 5 | APS-Lösung | 100,00 µl |
| 6 | Zellsuspension | 5,00 ml |

Die Herstellung des Immobilisats erfolgte wie bei Ansatz 1 beschrieben.

Dabei entstand ein festes, etwa 1 mm dickes Gel, das aber gegenüber Scherbelastungen, wie sie z.B. durch Rührung oder einfaches Biegen entstehen, sehr empfindlich war.

In den Ansätzen 1 bis 3 wurde das Verhältnis von Acrylamid zu Bisacrylamid variiert. Dabei lag im Ansatz 1 das Verhältnis von Acrylamid zu Bisacrylamid bei 30:1, im Ansatz 2 bei 19:0,8 und im Ansatz 3 bei 9:1.

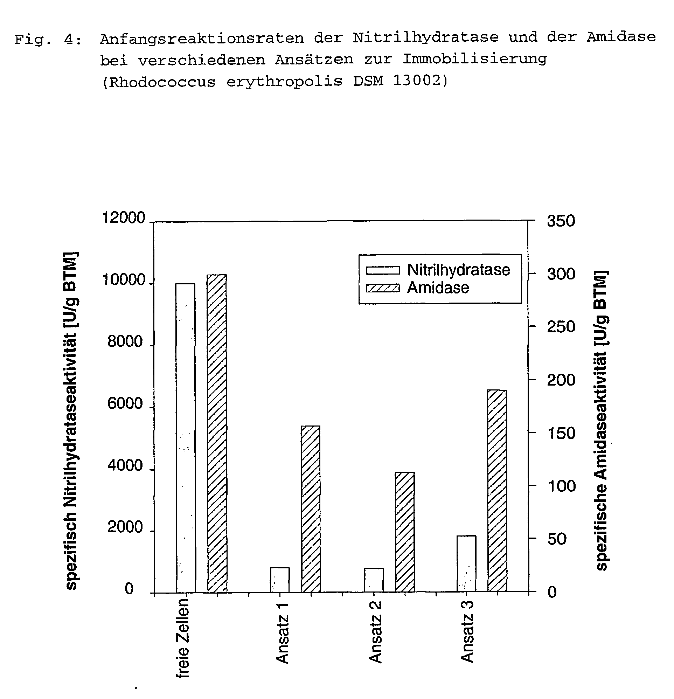

Die Anfangsreaktionsraten für die Nitrilhydratase und die Amidase bei den verschiedenen Ansätzen zur Immobilisierung der Biomasse sind in Fig. 4 dargestellt.

Anfangsreaktionsraten der Nitrilhydratase und der Amidase bei verschiedenen Acrylamid/Bisacrylamid-Konzentrationen in den Ansätzen zu Acrylamidimmobilisierung der ruhenden Zellen. Das Immobilisat wurde im Standardaktivitätstest eingesetzt und die Anfangsreaktionsraten bestimmt.

Es war eine Verbesserung der Ausbeute bezüglich der Nitrilhydrataseaktivität durch die Erniedrigung des Acrylamidanteils in der Polymerlösung festzustellen.

Beispiel 9: Induktion der Nitrilhydratase und Amidase Reaktion für Erythropolis rhodococcus 13002

Dazu wurde Medium 1 ohne β-Caprolactam und Kobaltchlorid angesetzt und die jeweiligen Komponenten in den weiteren Ansätzen zugegeben. Die Biomasse wurde nach der Aberntung im Standardaktivitätstest auf ihre spezifische Aktivität hin untersucht. Die erhaltenen Werte sind in folgender Tabelle dargestellt.

Einfluß von verschiedenen Induktoren auf die spezifische Aktivität der Biomasse

| Induktor | Nitrilhydrataseaktivität [U/g BTM] | Amidaseaktivität [U/g BTM] |
|---|---|---|
| ohne | 37854 | 255 |
| 0,50 g/l β-Caprolactam | 32159 | 136 |
| 1,00 mM Phenylpropionitril | 26515 | 172 |
| 1,00 g/l Acetamid | 1292 | 0 |
| 1,00 g/l Propionamid | 5712 | 124 |
| 1,00 g/l Harnstoff | 3070 | 0 |
| 0,01 g/l Kobaltchlorid | 37342 | 133 |
| 0,10 g/l Kobaltchlorid | 10815 | 170 |

Das Enzymsystem liegt in dem verwendeten Medium mit hoher Aktivität vor. Die Zugabe von β-Caprolactam erniedrigt die spezifische Aktivität der Amidase im Vergleich zu dem Medium ohne Induktoren. Auch durch Phenylpropionitril wurden beide Enzyme in ihrer Aktivität gehemmt. Propionamid, Acetamid und Harnstoff inhibieren die Aktivität der Nitrilhydratase gegenüber dem Ansatz ohne Induktoren um bis zu 90 %, die der Amidase um bis zu 50 %. Die Zugabe von Kobaltchlorid in dem vorgeschlagenen Medium führte bei geringen Konzentrationen zu einer Erniedrigung der spezifischen Amidaseaktivität, bei höheren Konzentrationen auch zu einem Verlust an spezifischer Aktivität der Nitrilhydratase.

Beispiel 10: Isolierung des Nitrilhydratase-Amidase-Komplexes

Für den Zellaufschluß wurde Rhodococcus erythropolis in Medium 2 (s. Bsp. 2) über Nacht (20 h, 28°C, 130 rpm) angezogen und anschließend geerntet (5000 x g, 15 min., 4°C). Das Zellpellet wurde in EDTA-Sucrose-Puffer aufgenommen und die Zellwand mit 1 mg/ml Lysozym abgebaut (Inkubation 1 h, 4°C).

EDTA-Sucrose-Puffer:

| | |
|---|---|
| 5 ml | 0,25 M EDTA pH 8 |
| 1 ml | 10 mM Tris/Cl pH 8 |
| 20 ml | 1 mM EDTA, 10 mM Tris/Cl pH 8, 34 % Sucrose |
| 30 mg | Lysozym |

Nach Zentrifugation und Waschen der Zellen (5000 x g, 15 min, 4°C) wird das Pellet in 20 g/l $KH_2PO_4$-Puffer (pH 7, + 10 mM Dithiotreitol) aufgenommen und mit der French Press (1250 kg/cm$^2$) dreimal unter Kühlung aufgeschlossen. Die Membranfraktion wurde durch Zentrifugation (20 000 x g, 30 min, 4°C) abgetrennt. Der so erhaltene Überstand enthält den Nitrilhydratase-Amidase-Komplex. Er enthält 10 % Restaktivität bezogen auf die eingesetzte Biofeuchtmasse. Alternativ lassen sich die Zellen direkt ohne Lysozymbehandlung nach Anzucht in einer Glasmühle (Braun Melsungen) unter N2-Kühlung aufschließen (3 x 2 nm, 30 sec, + 10 sec Kühlung). Die so erhaltene Restaktivität ist jedoch in der Regel deutlich geringer.

Beispiel 11: Abbau von Nitrilen durch Rhodococcus erythropolis DSM 13002, DSM 13475 und DSM 13476

Zur Untersuchung des Abbaus von Nitrilen durch die erfindungsgemäßen Mikroorganismen wurden die Stämme in Medium 1 (siehe Beispiel 2) über Nacht kultiviert (28°C, 150 rpm, 20 h) unter Zusatz von 5 g/l Acetonitril. Die Anzuchtmedium wurde abzentrifugiert, die Mikroorganismen wurden in Reaktionspuffer 1 (50 mM $Na_2HPO_4$, 50 mM $NaH_2PO_4$, pH 7,2) resuspendiert und die Reaktionen durch Zusatz der entsprechenden Nitrile gestartet. Die Reaktionstemperatur lag bei 22°C. Nach 1 bzw. 15 h wurde die Reaktion gestoppt und entweder über die Bestimmung der Substrate oder über die Bestimmung der Produkte wurde die Umsatzrate [(entstandenes Produkt/ eingesetztem Substrat) x 100] ermittelt. Folgende Tabelle zeigt die Ergebnisse für die 3 Mikroorganismen.

Umsatzraten in [%]

| Nitril | DSM 13002 | DSM 13475 | DSM 13476 |
|---|---|---|---|
| Acetonitril | | | |
| (1 h) | 56 | 100 | 46 |
| (15 h) | 77 | 100 | 96 |
| Adipinsäurenitril | 100 | 77 | 40 |
| (1 h) | 100 | 95 | 100 |
| (15 h) | | | |
| Benzonitril | | | |
| (1 h) | 5 | 5 | 5 |
| (15 h) | 65 | 46 | 35 |
| Acrylnitril | | | |
| (1 h) | 35 | n.b. | 45 |
| (15 h) | 42 | | 71 |
| Tricyanohexan | | | |
| (1 h) | 47 | 18 | 66 |
| (15 h) | 78 | 30 | 100 |
| n.b. nicht bestimmt | | | |

**Patentansprüche**

1. Verfahren zur enzymatischen Umsetzung von Verbindungen mit mindestens einer Nitrilfunktion und/oder mindestens einer Amidfunktion mit mindestens einem Mikroorganismus und/oder mindestens einem aus diesem Mikroorganismus isolierten Nitrilhydratase-Amidase-Komplex, **dadurch gekennzeichnet, daß** der Mikroorganismus ausgewählt ist aus der Gruppe, die gebildet wird von Rhodococcus erythropolis DSM 13002, Rhodococcus erythropolis DSM 13475 und Rhodococcus erythropolis DSM 13476.

**2.** Verfahren nach Anspruch 1, **dadurch gekennzeichnet, daß** der Mikroorganismus in Form ganzer Zellen vorliegt.

**3.** Verfahren nach Anspruch 1, **dadurch gekennzeichnet, daß** der Mikroorganismus und/oder der Nitrilhydratase-Amidase-Komplex in immobilisierter Form vorliegt.

**4.** Verfahren nach Anspruch 3, **dadurch gekennzeichnet, daß** der Mikroorganismus und/oder der Nitrilhydratase-Amidase-Komplex in Form von Alginatpolymeren immobilisiert vorliegt.

**5.** Verfahren nach den Ansprüchen 1 bis 4, **dadurch gekennzeichnet, daß** man als Verbindung mit mindestens einer Nitrilfunktion Verbindungen einsetzt, die ausgewählt sind aus der Gruppe, die gebildet wird von gesättigten aliphatischen Nitrilen, ethylenisch ungesättigten aliphatischen Nitrilen, aromatischen Nitrilen und heterocyclischen Nitrilen.

**6.** Verfahren nach Anspruch 5, **dadurch gekennzeichnet, daß** man Propionitril, Acetonitril und/oder Acrylnitril einsetzt.

**7.** Verfahren nach den Ansprüchen 1 bis 4, **dadurch gekennzeichnet, daß** man als Verbindung mit mindestens einer Amidfunktion Verbindungen einsetzt, die ausgewählt sind aus der Gruppe, die gebildet wird von gesättigten aliphatischen Amiden, ethylenisch ungesättigten aliphatischen Amiden, aromatischen Amiden.und heterocyclischen Amiden.

**8.** Verfahren nach Anspruch 7, **dadurch gekennzeichnet, daß** man Propioamid, Acetoamid und/oder Acrylamid einsetzt.

**9.** Verfahren nach den Ansprüchen 1 bis 8, **dadurch gekennzeichnet, daß** man das Verfahren als absatzweise Prozeßführung mit kontinuierlicher Substratzugabe durchführt.

**10.** Rhodococcus erythropolis DSM 13002.

**11.** Rhodococcus erythropolis DSM 13475.

**12.** Rhodococcus erythropolis DSM 13476.

**13.** Nitrilhydratase-Amidase-Komplex aus einem Mikroorganismus, der ausgewählt ist aus der Gruppe, die gebildet wird von Rhodococcus erythropolis DMS 13002, Rhodococcus erythropolis DSM 13475 und Rhodococcus erythropolis DSM 13476.

## Claims

**1.** A process for the enzymatic conversion of compounds having at least one nitrile function and/or at least one amide function using at least one microorganism and/or at least one nitrile hydratase-amidase complex isolated from said microorganism, which comprises selecting the microorganism from the group formed by Rhodococcus erythropolis DSM 13002, Rhodococcus erythropolis DSM 13475 and Rhodococcus erythropolis DSM 13476.

**2.** The process according to claim 1, wherein the microorganism is present in the form of whole cells.

**3.** The process according to claim 1, wherein the microorganism and/or the nitrile hydratase-amidase complex is present in immobilized form.

**4.** The process according to claim 3, wherein the microorganism and/or the nitrile hydratase-amidase complex is present immobilized in the form of alginate polymers.

**5.** The process according to claims 1 to 4, wherein compounds selected from the group formed by saturated aliphatic nitriles, ethylenically unsaturated aliphatic nitriles, aromatic nitriles and heterocyclic nitriles are used as compound having at least one nitrile function.

**6.** The process according to claim 5, wherein propionitrile, acetonitrile and/or acrylonitrile are used.

**7.** The process according to claims 1 to 4, wherein compounds selected from the group formed by saturated aliphatic

amides, ethylenically unsaturated aliphatic amides, aromatic amides and heterocyclic amides are used as compound having at least one amide function.

8. The process according to claim 7, wherein propioamide, acetamide and/or acrylamide are used.

9. The process according to claims 1 to 8, which is carried out as batchwise process with continuous substrate addition.

10. A Rhodococcus erythropolis DSM 13002.

11. A Rhodococcus erythropolis DSM 13475.

12. A Rhodococcus erythropolis DSM 13476.

13. A nitrile hydratase-amidase complex from a microorganism selected from the group formed by Rhodococcus erythropolis DSM 13002, Rhodococcus erythropolis DSM 13475 and Rhodococcus erythropolis DSM 13476.

**Revendications**

1. Procédé de mise en réaction enzymatique de composés comportant au moins une fonction nitrile et/ou au moins une fonction amide avec au moins un micro-organisme et/ou un complexe nitrilhydratase-amidase isolé de ce micro-organisme, **caractérisé en ce que** le micro-organisme est choisi parmi le groupe qui est formé de Rhodococcus erythropolis DSM 13002, de Rhodococcus erythropolis DSM 13475 et de Rhodococcus erythropolis DSM 13476.

2. Procédé suivant la revendication 1, **caractérisé en ce que** le micro-organisme se présente sous la forme de cellules entières.

3. Procédé suivant la revendication 1, **caractérisé en ce que** le micro-organisme et/ou le complexe de nitrilhydratase-amidase se présentent sous une forme immobilisée.

4. Procédé suivant la revendication 3, **caractérisé en ce que** le micro-organisme et/ou le complexe de nitrilhydratase-amidase se présentent sous une forme immobilisée à des polymères d'alginate.

5. Procédé suivant les revendications 1 à 4, **caractérisé en ce que**, comme composé présentant au moins une fonction nitrile, on met en oeuvre des composés qui sont choisis parmi le groupe qui est formé des nitriles aliphatiques saturés, des nitriles aliphatiques éthyléniquement insaturés, des nitriles aromatiques et des nitriles hétérocycliques.

6. Procédé suivant la revendication 5, **caractérisé en ce qu'**on met en oeuvre du propionitrile, de l'acétonitrile et/ou de l'acrylonitrile.

7. Procédé suivant les revendications 1 à 4, **caractérisé en ce que**, comme composé présentant au moins une fonction amide, on met en oeuvre des composés qui sont choisis parmi le groupe qui est formé des amides aliphatiques saturés, des amides aliphatiques éthyléniquement insaturés, des amides aromatiques et des amides hétérocycliques.

8. Procédé suivant la revendication 7, **caractérisé en ce qu'**on met en oeuvre du propionamide, de l'acétamide et/ou de l'acrylamide.

9. Procédé suivant les revendications 1 à 8, **caractérisé en ce qu'**on effectue le procédé avec une conduite discontinue du processus et avec addition continue de substrat.

10. Rhodococcus erythropolis DSM 13002.

11. Rhodococcus erythropolis DSM 13475.

12. Rhodococcus erythropolis DSM 13476.

13. Complexe de nitrilhydratase-amidase issu d'un micro-organisme qui est choisi parmi le groupe qui est formé de

Rhodococcus erythropolis DSM 13002, de Rhodococcus erythropolis DSM 13475 et de Rhodococcus erythropolis DSM 13476.

Fig. 1: Aktivitätsbestimmung
(Rhodococcus erythropolis DSM 13002)

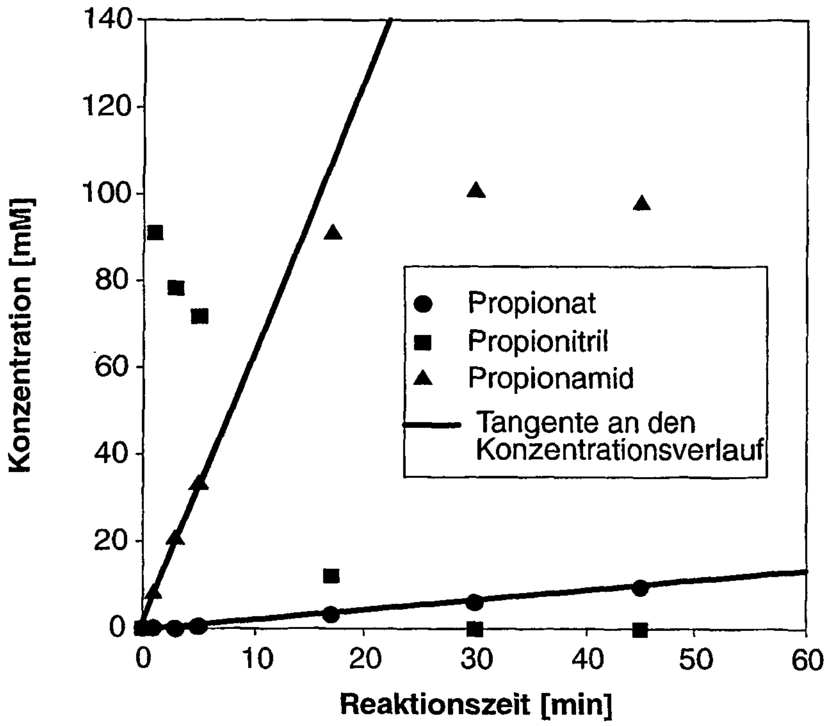

Fig. 2: Temperatur Optimum der Enzymreaktion (Rhodococcus erythropolis DSM 13002)

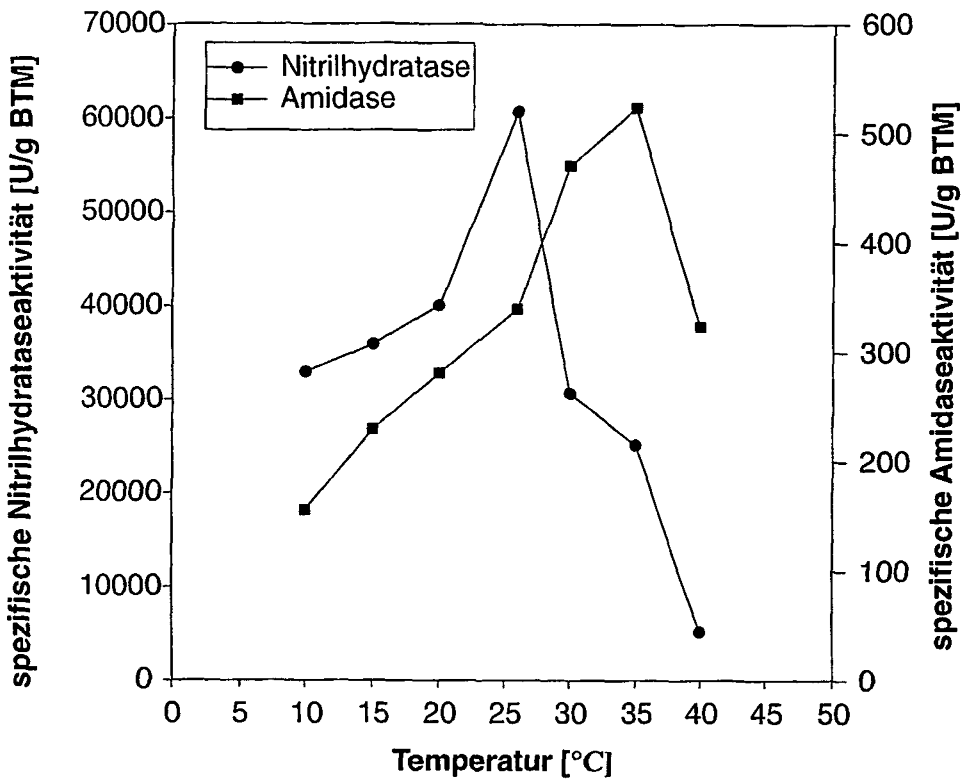

Fig. 3: pH-Optimum der Enzymreaktion
(Rhodococcus erythropolis DSM 13002)

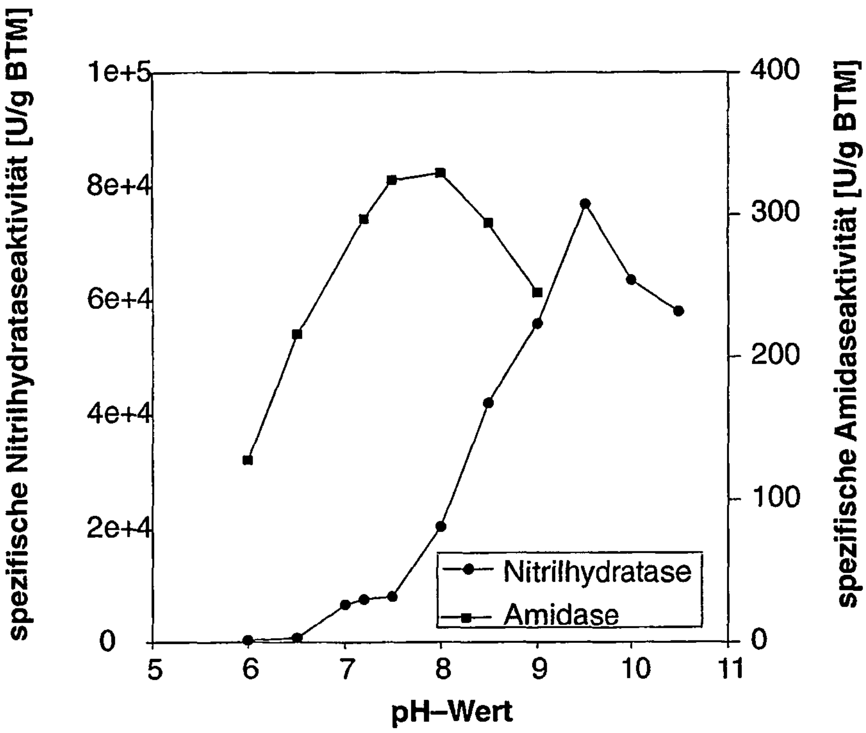

Fig. 4: Anfangsreaktionsraten der Nitrilhydratase und der Amidase bei verschiedenen Ansätzen zur Immobilisierung (Rhodococcus erythropolis DSM 13002)